# FASCICULE DE BREVET EUROPEEN

(11) **EP 4 396 168 B1**
(45) Date de publication et mention de la délivrance du brevet: **01.10.2025**
(21) Numéro de dépôt: 22773751.7
(22) Date de dépôt: 30.08.2022
(51) Int. Cl.: C07D 259/00, C06B 43/00

(54) **PROCEDE DE FABRICATION DE L'ANION PENTAZOLATE A L'AIDE D'UN OXYDANT A L'IODE HYPERVALENT**
VERFAHREN ZUR HERSTELLUNG DES PENTAZOLATANIONS UNTER VERWENDUNG EINES HYPERVALENTEN IODOXIDANS
METHOD FOR PRODUCING THE PENTAZOLATE ANION USING A HYPERVALENT IODINE OXIDANT

(30) Priorité: 03.09.2021 FR 2109197
(43) Date de publication de la demande: 10.07.2024
(73) Titulaire: ArianeGroup SAS, 78130 Les Mureaux (FR); Centre National de la Recherche Scientifique, 75794 Paris Cedex 16 (FR); UNIVERSITE CLAUDE BERNARD - LYON 1, 69622 Villeurbanne Cédex (FR)
(72) Inventeur: JOUCLA, Lionel, 91710 Vert-le-Petit (FR); GASNIER, Gwénaël, 91710 Vert-le-Petit (FR); COMTE, Sébastien, 91710 Vert-le-Petit (FR); SILVA COSTA, Raphaël, 91710 Vert-le-Petit (FR)
(74) Mandataire: Cabinet Beau de Loménie
(86) Numéro de dépôt international: PCT/FR2022/051632
(87) Numéro de publication internationale: WO 2023/031552

(56) Documents cités:
- CN-A- 106 518 797
- CN-A- 106 748 602
- CN-A- 107 365 277
- XU YUANGANG ET AL: "A series of energetic metal pentazolate hydrates", vol. 549, no. 7670, 7 September 2017 (2017-09-07), London, pages 78 - 81, XP055901398, ISSN: 0028-0836, Retrieved from the Internet <URL:https://www.nature.com/articles/nature23662.pdf> DOI: 10.1038/nature23662
- ZHANG CHONG ET AL: "Synthesis and characterization of the pentazolate anion cyclo -N5- in (N5)6(H3O)3(NH4)4Cl", vol. 355, no. 6323, 27 January 2017 (2017-01-27), US, pages 374 - 376, XP055901462, ISSN: 0036-8075, Retrieved from the Internet <URL:http://dx.doi.org/10.1126/science.aah3840> DOI: 10.1126/science.aah3840

## Description

### Domaine Technique

La présente invention propose un procédé de fabrication de l'anion pentazolate (cyclo-N₅⁻) mettant en œuvre un oxydant à l'iode hypervalent. L'anion pentazolate a la formule reproduite ci-dessous.

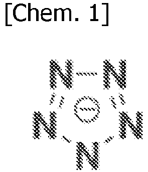

La présente invention concerne également la préparation d'une composition énergétique à partir de l'anion pentazolate obtenu.

### Technique antérieure

La recherche de nouvelles charges énergétiques, oxydantes ou explosives, a été réalisée jusqu'à présent par la fonctionnalisation de structures carbonées avec des groupements explosophores, par exemple du type azoture (N₃), nitrates (O-NO₂) ou nitramines (N-NO₂). Cette approche atteint aujourd'hui un plafond en termes d'énergie interne. Il est donc souhaitable de trouver de nouvelles molécules à haute densité énergétique. A ce titre, l'anion pentazolate a été identifié dans la littérature comme un composé prometteur, aussi bien pour des applications civiles de propulsion que militaires. La préparation de l'anion pentazolate a été décrite pour la première fois en 2017 par le groupe de Zhang [Science, 2017, 355, 374-376]. Dans ces travaux, il a été proposé de réaliser une oxydation d'un arylpentazole phénolique à l'aide d'un système oxydant à base d'un dérivé organométallique au fer(II) et d'acide métachloroperbenzoïque (m-CPBA) en excès. La mise en œuvre du peracide m-CPBA pose néanmoins des problèmes de sécurité et met en œuvre une purification relativement complexe par chromatographie sur gel de silice. Il est également souhaitable d'améliorer le caractère reproductible de cette synthèse ainsi que les rendements obtenus qui sont de l'ordre de 5% à 15%.

La préparation de l'anion pentazolate a été aussi décrite dans CN 107365277, CN 106518797, CN 106748602, et dans Nature, 2017, 549, pages 78-81.

### Exposé de l'invention

L'invention concerne un procédé de fabrication de l'anion pentazolate, comprenant au moins :
- une oxydation d'un arylpentazole phénolique par un oxydant à l'iode hypervalent en présence d'une base,
   ledit oxydant à l'iode hypervalent étant de formule générale A-B où le groupement A désigne un cycle benzénique, et le groupement B est une structure comprenant un atome d'iode hypervalent ayant l'une des deux formules B1 ou B2 ci-dessous : 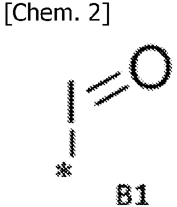 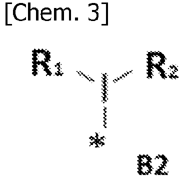
   avec *- désignant dans les formules B1 et B2 la liaison de l'atome d'iode hypervalent au cycle benzénique A,
   dans la formule B2, R₁ et R₂ étant identiques ou différents et choisis indépendamment l'un de l'autre parmi : **-OCOR, **-OR, où R désigne une chaîne alkyle comprenant entre 1 et 4 atomes de carbone, linéaire ou ramifiée, ou R₁ étant **-OH et R₂ étant **-OTs avec Ts désignant un groupement tosyle, où **- désigne la liaison de l'atome d'oxygène à l'atome d'iode hypervalent.

Dans la suite l'expression « oxydant à l'iode hypervalent » sera désignée par « oxydant » pour des raisons de concision.

La base permet de déprotoner la fonction -OH de l'arylpentazole phénolique afin que l'oxydation se produise. Dans la formule B2, R₁ et R₂ sont avantageusement identiques.

L'invention est remarquable en ce qu'elle propose d'utiliser un oxydant particulier pour obtenir l'anion pentazolate à partir d'un arylpentazole phénolique, en s'affranchissant en particulier de l'emploi du m-CPBA de l'art antérieur. L'invention fournit ainsi une synthèse plus sécuritaire, ainsi que des rendements reproductibles. L'oxydant mis en œuvre permet également de simplifier la purification par rapport à l'art antérieur en rendant possible une simple extraction liquide-liquide pour éliminer les restes d'oxydant, ce qui permet d'envisager une montée en échelle de production.

Dans un exemple de réalisation, l'oxydation est réalisée dans un solvant comprenant l'hexafluoroisopropanol (HFIP) et au moins un composé choisi parmi les alcools ou l'acétonitrile.

L'emploi de ce solvant permet d'améliorer le rendement de formation de l'anion pentazolate car il facilite la dépolymérisation de l'oxydant, activant ainsi l'oxydation de l'arylpentazole phénolique.

On peut en particulier réaliser l'oxydation dans un solvant comprenant l'hexafluoroisopropanol et du méthanol (MeOH).

On peut plus généralement réaliser l'oxydation dans un solvant perfluoré qui peut être différent du HFIP comme dans le trifluoroéthanol (TFE). D'une manière générale, l'oxydation peut être réalisée dans un mélange d'un alcool, tel que le méthanol (MeOH), et de solvant perfluoré ou d'acétonitrile.

Dans un exemple de réalisation, le groupement B est de formule B1.

Ce type d'oxydant permet de limiter la génération de sels contaminants par rapport à l'emploi du groupement de formule B2, simplifiant davantage encore l'isolation et la purification de l'anion pentazolate obtenu.

Dans ce cas, l'oxydant peut en particulier être l'iodosylbenzène (PhIO) dont la formule est fournie ci-dessous.

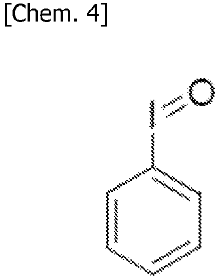

Dans un exemple de réalisation, le groupement B est de formule B1 et l'oxydation est réalisée dans un solvant comprenant l'hexafluoroisopropanol et au moins un composé choisi parmi les alcools ou l'acétonitrile.

Cette caractéristique permet avantageusement d'obtenir un rendement de formation de l'anion pentazolate particulièrement élevé par rapport aux rendements des techniques de l'art antérieur, pouvant aller jusqu'à plus de 50%.

En variante, le groupement B est de formule B2 et peut présenter la formule générale ci-dessous.

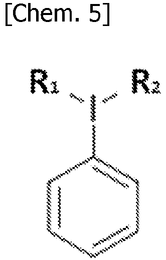

La chaîne alkyle R qui peut faire partie intégrante des substituants R₁ et R₂ décrite plus haut peut être non substituée.

Selon un exemple, le groupement B est de formule B2 avec R₁ et R₂ identiques et désignant chacun **-OCOMe ou **-OMe, où Me désigne un groupement méthyle. En particulier, l'oxydant peut être le diacétate d'iodobenzène (PIDA) dont la formule est fournie ci-dessous.

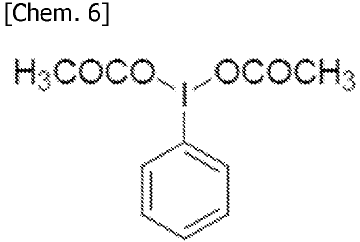

D'autres groupements de formule B2 sont envisageables avec par exemple R₁ et R₂ identiques et désignant chacun **-OCOtBu, où *t*Bu désigne un groupement tertiobutyle.

Selon une autre variante, on peut utiliser le réactif de Koser comme oxydant, dont la formule est fournie ci-dessous, avec Ts correspondant au groupement tosyle :

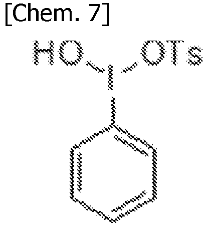

Quelle que soit sa structure, l'oxydant peut être obtenu, par des techniques connues en soi, par exemple à partir de l'halogénure d'aryle correspondant ou d'un dérivé aromatique simple. Les deux équations ci-dessous illustrent, dans le cadre d'un exemple, ces deux voies de synthèse pour la formation de l'oxydant PIDA.

[Chem. 8] Voie 1. C₆H₅I + CH₃CO₃H + CH₃CO₂H → C₆H₅I(O₂CCH₃)₂ + H₂O Voie 2: C₆H₆ + I₂ + 2 CH₃CO₂H + K₂S₂O₈ → C₆H₅I(O₂CCH₃)₂ + KI + H₂SO₄ + KHSO₄

Dans un exemple de réalisation, la base comprend au moins un composé choisi parmi : un hydroxyde alcalin, un hydroxyde alcalino-terreux, un hydroxyde d'un métal, l'hydroxyde d'ammonium, un hydroxyde d'ammonium quaternaire, un carbonate alcalin ou un mélange de ces composés.

L'emploi d'une base comprenant un ion hydroxyde est avantageuse car elle limite la génération de produits contaminants par rapport à l'emploi de bases du type carbonates alcalins. L'emploi de bases carbonates demeure néanmoins dans le cadre de l'invention.

En particulier, la base peut être l'hydroxyde de sodium (NaOH). En variante ou en combinaison, la base peut comprendre de l'hydroxyde de lithium (LiOH), de l'hydroxyde de potassium (KOH), de l'hydroxyde de calcium Ca(OH)₂, de l'hydroxyde de magnésium Mg(OH)₂, de l'hydroxyde d'aluminium Al(OH)₃, ou de l'hydroxyde de baryum (Ba(OH)₂).

L'oxydation peut être réalisée par mise en œuvre des étapes successives suivantes :
- dissolution de la base dans un alcool, par exemple dans le méthanol, à une première température comprise entre 10°C et 30°C, par exemple à température ambiante (20°C),
- ajout d'un solvant perfluoré, par exemple HFIP ou TFE, et/ou d'acétonitrile, cet ajout étant effectué à la première température,
- refroidissement du mélange à une deuxième température inférieure à la première température et comprise entre -60°C et -20°C, par exemple égale à -40°C,
- ajout, à la deuxième température, de l'arylpentazole phénolique et de l'oxydant, l'oxydant étant ajouté par portions,
- agitation du mélange obtenu à la deuxième température pendant une durée comprise entre 8 heures et 48 heures, par exemple pendant 24 heures,
- remontée progressive de la température du mélange, par exemple jusqu'à la première température, avec au moins un palier à une troisième température supérieure à la deuxième température et inférieure à la première température, la troisième température étant comprise entre -40°C et 0°C, par exemple égale à - 20°C, et maintien à cette troisième température pendant une durée comprise entre 8 heures et 48 heures, par exemple 24 heures, et éventuellement avec un palier supplémentaire à une quatrième température supérieure à la troisième température et inférieure à la première température, la quatrième température étant comprise entre -20°C et 20°C, par exemple à 0°C, et maintien à cette quatrième température pendant une durée comprise entre 8 heures et 48 heures, par exemple 24 heures.

L'oxydant est présent à raison d'au moins un équivalent, par exemple entre un et cinq équivalents, voire entre deux et quatre équivalents, préférentiellement environ 3 équivalents, par rapport à l' arylpentazole phénolique. La base est présente à raison d'au moins un équivalent, par exemple entre un et cinq équivalents, par rapport à l'arylpentazole phénolique. Les quantités relatives sont prises avant le début de l'oxydation.

L'arylpentazole phénolique peut être obtenu par des techniques connues en soi. Il a la structure chimique suivante :

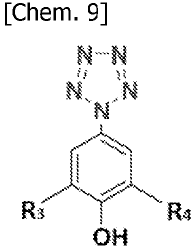

Dans la formule ci-dessus, R₃ et R₄ sont identiques ou différents et choisis indépendamment l'un de l'autre parmi : les chaînes alkyles comprenant entre 1 et 4 atomes de carbone, linéaires ou ramifiées, non substituées ou substituées par un groupement alcoxy en C₁ ou C₂ ou par une dialkylamine. R₃ et R₄ sont de préférence non substitués. R₃ et R₄ peuvent être chacun un groupement méthyle ou éthyle.

Dans un exemple de réalisation, l'oxydation est réalisée dans un solvant et le procédé comprend en outre successivement :
- une élimination du solvant,
- une extraction liquide-liquide, à l'aide d'un solvant d'extraction, pour éliminer les restes d'oxydant,
- une élimination du solvant d'extraction, et
- une extraction sélective de l'anion pentazolate après cette élimination, l'anion pentazolate étant sélectivement extrait dans un milieu liquide comprenant l'un au moins de l'éthanol, l'acétonitrile ou l'acétone, ou un mélange de ces composés.

L'invention permet avantageusement une séparation simple de l'anion pentazolate des autres composés présents, tels que le chlorure de sodium (NaCl) qui peut provenir de l'étape de génération de l'arylpentazole phénolique précédant l'oxydation. Cette extraction sélective peut être réalisée à température ambiante (20°C). On peut de préférence utiliser l'acétonitrile et/ou l'acétone pour réaliser cette extraction sélective.

L'invention vise également un procédé de fabrication d'une composition énergétique, comprenant au moins la fabrication de l'anion pentazolate par le procédé décrit plus haut, et le mélange de l'anion pentazolate ainsi fabriqué à un liant pour obtenir la composition énergétique.

Brève description des dessins
[Fig. 1] La figure 1 est un résultat d'analyse par chromatographie ionique réalisé après mise en œuvre d'un exemple de procédé selon l'invention, cette figure met en évidence la présence de l'anion pentazolate.
[Fig. 2] La figure 2 est un résultat d'analyse par RMN ¹⁴N avec étalonnage externe au NH₄Cl réalisé après mise en œuvre d'un exemple de procédé selon l'invention.
[Fig. 3] La figure 3 est un résultat d'analyse par calorimétrie différentielle à balayage réalisé après mise en œuvre d'un exemple de procédé selon l'invention.

### Exemples

### Préparation du précurseur du type arylpentazole (réduction catalytique du 2,6-diméthyl-4-nitrophénol)

Dans un tricol de 1 L, le palladium sur charbon (Pd/C, 10% massique, 240 mg) est mis en suspension dans 44 mL d'eau distillée puis le système est placé sous un balayage d'argon. Une solution formée de borohydrure de sodium (NaBH₄) (4,54 g, 120 mmol) dans 100 mL d'une solution 0,1 N d'hydroxyde de sodium (NaOH) est ajoutée goutte à goutte (pendant environ 20 minutes) à température ambiante (20°C). Une solution formée de 2,6-diméthyl-4-nitrophénol (10,00 g, 60 mmol) dans 360 mL d'une solution aqueuse NaOH 1N (solution chauffée à 40°C pour faciliter la solubilisation) est alors ajoutée au goutte à goutte (pendant environ 60 minutes). Le milieu réactionnel est agité à température ambiante pendant 4 heures puis filtré sur célite (lavages avec de l'eau). Une solution aqueuse d'acide chlorhydrique (HCl) 3N (400 mL) est ajoutée au filtrat puis ce dernier est lavé avec 3*100 mL d'acétate d'éthyle (AcOEt). La phase aqueuse est concentrée (~ 80 mL) puis 100 mL d'une solution HCl 37% sont ajoutés. La solution est placée au réfrigérateur une nuit puis le précipité est filtré. Ce dernier est dissous dans 200 mL d'eau distillée puis cette solution est neutralisée par ajout d'hydrogénocarbonate de sodium (NaHCO₃) jusqu'à pH~7/8. La phase aqueuse est extraite par 3*100 mL d'AcOEt puis les phases organiques réunies sont séchées sur du sulfate de magnésium (MgSO₄), filtrées et évaporées à sec. Le produit est obtenu sous la forme d'un solide violet correspondant à la 3,5-diméthyl-4-hydroxyaniline (7,35 g, 91%).

### Préparation du précurseur du type arylpentazole (diazotation puis azoturation)

Dans un tricol de 250mL, la 3,5-diméthyl-4-hydroxyaniline (3,00g, 22mmol, 1 éq.) est dissoute dans 17mL de tétrahydrofurane (THF) puis la solution est refroidie à - 5°C. On ajoute une solution de HCl 37% (3,25mL, 40mmol, 1,8éq.) au goutte à goutte en maîtrisant l'exothermie. Une solution de nitrite de sodium (NaNO₂) (1,59 g, 23mmol, 1,05 éq.) dans 8 mL d'un mélange MeOH/H₂O (1/1 v/v) est alors ajoutée au goutte à goutte en conservant la température du milieu réactionnel en dessous de -2°C. Après la fin de l'ajout, le milieu réactionnel est agité à -5°C pendant 30 minutes puis refroidi à -40°C. On ajoute 75 mL d'un mélange MeOH/heptane (1/2 v/v) préalablement refroidi à -40°C en une fois puis on augmente l'agitation pour créer une émulsion. Une solution préalablement refroidie à -40°C d'azoture de sodium (NaN₃) (1,50g, 23 mmol, 1 éq.) dans 8 mL d'un mélange MeOH/H₂O (1/3 v/v) est alors ajoutée au goutte à goutte. Après 15 minutes d'agitation à -40°C, la solution est filtrée sur un fritté double enveloppe maintenu à -40°C. Le produit arylpentazole phénolique est obtenu sous la forme d'un solide violet et conservé dans un flacon plastique à -196°C. Une analyse par RMN ¹H (400MHz, CD₃OD) montre généralement un ratio ArN₅/ArN₃ d'environ 9/1.

### Oxydation pour la génération de l'anion pentazolate

A une solution de NaOH (0,44 g, 11 mmol) dans 30 mL de MeOH on ajoute 30 mL de hexafluoroisopropanol (HFIP) puis ce mélange est refroidi à -40°C. Le précurseur arylpentazole phénolique, conservé à -40°C, est alors ajouté en une fois. L'oxydant iodosylbenzène (PhIO) (2,42 g) est ajouté par portions puis le milieu réactionnel est agité 24 heures à -40°C puis laissé remonter lentement à température ambiante (20°C). Les solvants sont évaporés à sec puis le résidu est repris avec 100 mL d'un mélange H₂O/AcOEt (1/1 v/v). La phase aqueuse est lavée avec 3*20 mL d'AcOEt puis évaporée à sec (co-évaporation avec de l'éthanol). Le solide ainsi obtenu est analysé par spectrométrie de masse haute résolution (HRMS), ionisation chimique (CI) et RMN ¹⁴N pour confirmer la présence de l'anion pentazolate. Le taux massique moyen constaté est d'environ 5-10% en azote et le contaminant majoritaire est du NaCl.

Le schéma réactionnel ci-dessous résume le procédé de synthèse de l'anion pentazolate mis en œuvre dans cet exemple.

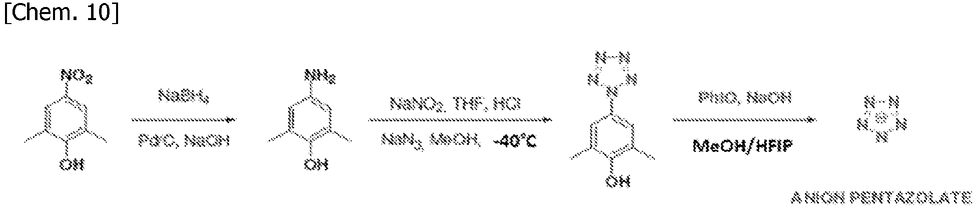

### Purification de l'anion pentazolate

Le solide obtenu précédemment après l'oxydation (~ 1 g) est mis en suspension dans 100 mL d'un solvant anhydre (acétonitrile ou acétone). Après agitation pendant 3 jours, le solide est filtré, lavé avec de petits volumes de solvant puis ce dernier est évaporé à sec. On récupère entre 50 et 80 mg d'un solide jaunâtre. L'analyse par RMN ¹⁴N permet de déterminer un taux massique d'environ 40 à 45% en anion pentazolate.

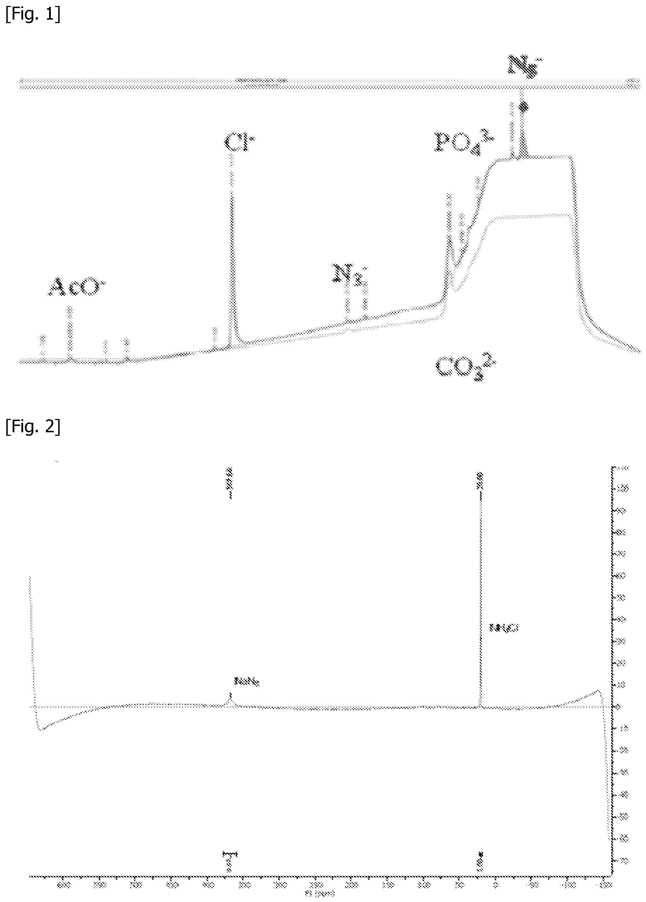

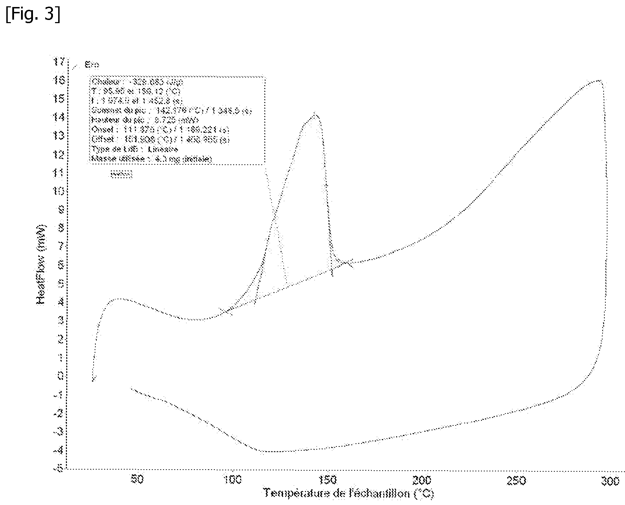

Les figures 1 à 3 fournissent les caractérisations physico-chimiques du produit obtenu et montrent la détection de l'anion pentazolate. La figure 1 est un résultat d'analyse par chromatographie ionique. La figure 2 est un résultat d'analyse par RMN ¹⁴N montrant un déplacement chimique caractéristique (δ 367 ppm vs NH₃, étalon NH₄Cl à δ 20 ppm). La figure 3 est un résultat d'analyse par calorimétrie différentielle à balayage (« DSC ») montrant une température de décomposition d'environ 110°C conforme à celle décrite dans la littérature pour les complexes NaN₅.xH₂O avec x compris entre 2 et 3.

D'autres essais ont été réalisés en faisant varier la nature de l'oxydant et du solvant lors de l'oxydation de l'arylpentazole phénolique et sont regroupés dans le tableau 1 ci-dessous (l'essai noté « Exp. 3 » dans le tableau ci-dessous correspond au mode opératoire qui vient d'être décrit). Un mode opératoire identique à celui décrit plus haut a été utilisé pour ces essais en remplaçant, selon les cas, HFIP par CH₃CN ou TFE, et PhIO par PIDA.

**[Table 1]**

| **Exp.** | **Oxydant** | **Solvant** | **Base** | **m(g) brut** | **% mass. N** | **N₅ (mmol)** | **Rendement (%)** |
|---|---|---|---|---|---|---|---|
| **1** | PIDA | MeOH - CH₃CN | NaOH | 0,32 | 2,8 | 0,13 | |
| **2** | PhIO | MeOH - TFE | NaOH | 1,74 | 1,3 | 0,32 | 8,7 |
| **3** | PhIO | MeOH - HFIP | NaOH | 1,76 | 7,8 | 1,93 | 52,5 |
| **4** | PIDA | MeOH - TFE | NaOH | 1,68 | 2,7 | 0,64 | 19,5 17,4 |
| **5** | PIDA | MeOH - HFIP | NaOH | 1,06 | 6,1 | 0,93 | 25,2 4,8 |
| **6** | PhIO | MeOH - HFIP | | 0,45 | 2,7 | 0,17 | |
| **7** | PhIO | MeOH - HFIP | NaOH | 1,73 1,73 | 6,3 | 1,55 | 34,8 |

Le tableau 1 montre l'efficacité des oxydants PhIO et PIDA dans différents solvants pour obtenir l'anion pentazolate. On peut noter que la suppression de la base (Exp. 6) inhibe la formation de l'espèce N₅⁻ et que la mise en œuvre de PhIO dans un solvant MeOH-HFIP en présence de NaOH donne un résultat optimal (Exp. 3) qui est reproductible (Exp. 7).

Une autre série d'essai a été réalisée en utilisant d'autres oxydants et bases. Les résultats sont regroupés dans le tableau 2 ci-dessous. Dans cette série d'essai, le mode opératoire pour l'oxydation est identique à celui décrit plus haut en remplaçant PhIO par PhI(OMe)₂ ou IBX, HFIP par CH₃CN ou TFE et NaOH par MeONa selon les cas.

**[Table 2]**

| **Exp.** | **Oxydant** | **Solvant** | **Base** | **m (g) brut** | **% mass.** N | **N₅ (mmol)** | **Rendement (%)** |
|---|---|---|---|---|---|---|---|
| **8** | PhI(OMe)₂ | MeOH - CH₃CN | NaOH | 2,39 | 1,1 | 0.39 | 10,5 |
| **9** | PhI(OMe)₂ | MeOH - TFE | NaOH | 1,07 | 2,6 | 0,40 | 11,1 |
| **10** | PhI(OMe)₂ | MeOH - HFIP | NaOH | 1,65 | 3,4 | 0,80 | 21,9 |
| **11** | PhI(OMe)₂ | MeOH -TFE | MeONa | 1,16 | 6,2 | 1,02 | 27,9 |
| **12** | IBX | MeOH - CH₃CN | NaOH | | | | - |

Le tableau 2 montre l'efficacité de l'oxydant PhI(OMe)₂ dans différents solvants afin de générer l'anion pentazolate. On note également la possibilité d'utiliser une base de type alcoolate (Exp. 11) et la totale inefficacité de l'IBX (Exp. 12) de formule fournie ci-dessous, un oxydant hors invention du type I(V) (alors que les oxydants PIDA, PhIO et PhI(OMe)₂ sont de type I(III)).

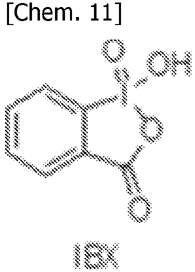

Un essai d'oxydation de l'arylpentazole phénolique en utilisant le réactif de Koser de formule rappelée ci-dessous a également été réalisé en utilisant une base hydroxyde d'ammonium et a conduit à un rendement de 25% pour l'étape d'oxydation.

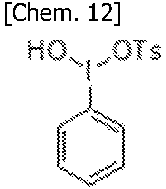

L'expression « compris(e) entre ... et ... » doit se comprendre comme incluant les bornes.

## Revendications

1. Procédé de fabrication de l'anion pentazolate, comprenant au moins :
- une oxydation d'un arylpentazole phénolique par un oxydant à l'iode hypervalent en présence d'une base,
ledit oxydant à l'iode hypervalent étant de formule générale A-B où le groupement A désigne un cycle benzénique, et le groupement B est une structure comprenant un atome d'iode hypervalent ayant l'une des deux formules B1 ou B2 ci-dessous : 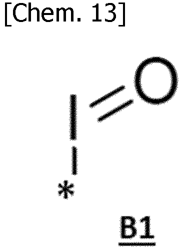 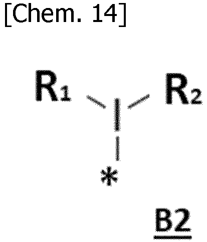
avec *- désignant dans les formules B1 et B2 la liaison de l'atome d'iode hypervalent au cycle benzénique A,
dans la formule B2, R₁ et R₂ étant identiques ou différents et choisis indépendamment l'un de l'autre parmi : **-OCOR, **-OR, où R désigne une chaîne alkyle comprenant entre 1 et 4 atomes de carbone, linéaire ou ramifiée, ou R₁ étant **-OH et R₂ étant **-OTs avec Ts désignant un groupement tosyle, où **- désigne la liaison de l'atome d'oxygène à l'atome d'iode hypervalent,
l'arylpentazole phénolique ayant la structure chimique suivante : 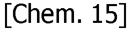 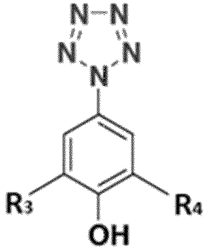
R₃ et R₄ étant identiques ou différents et choisis indépendamment l'un de l'autre parmi : les chaînes alkyles comprenant entre 1 et 4 atomes de carbone, linéaires ou ramifiées, non substituées ou substituées par un groupement alcoxy en C₁ ou C₂ ou par une dialkylamine.

2. Procédé selon la revendication 1, dans lequel l'oxydation est réalisée dans un solvant comprenant l'hexafluoroisopropanol et au moins un composé choisi parmi les alcools ou l'acétonitrile.

3. Procédé selon la revendication 1 ou 2, dans lequel le groupement B est de formule B1.

4. Procédé selon la revendication 3, dans lequel le groupement B est de formule B1 et l'oxydation est réalisée dans un solvant comprenant l'hexafluoroisopropanol et au moins un composé choisi parmi les alcools ou l'acétonitrile.

5. Procédé selon la revendication 1 ou 2, dans lequel le groupement B est de formule B2 avec R₁ et R₂ identiques et désignant chacun **-OCOMe ou **-OMe, où Me désigne un groupement méthyle.

6. Procédé selon l'une quelconque des revendications 1 à 5, dans lequel la base comprend au moins un composé choisi parmi : un hydroxyde alcalin, un hydroxyde alcalino-terreux, un hydroxyde d'un métal, l'hydroxyde d'ammonium, un hydroxyde d'ammonium quaternaire, un carbonate alcalin ou un mélange de ces composés.

7. Procédé selon la revendication 6, dans lequel la base est l'hydroxyde de sodium.

8. Procédé selon l'une quelconque des revendications 1 à 7, dans lequel l'oxydation est réalisée dans un solvant et dans lequel le procédé comprend en outre successivement :
- une élimination du solvant,
- une extraction liquide-liquide, à l'aide d'un solvant d'extraction, pour éliminer les restes d'oxydant,
- une élimination du solvant d'extraction, et
- une extraction sélective de l'anion pentazolate après cette élimination, l'anion pentazolate étant sélectivement extrait dans un milieu liquide comprenant l'un au moins de l'éthanol, l'acétonitrile ou l'acétone, ou un mélange de ces composés.

9. Procédé de fabrication d'une composition énergétique, comprenant au moins la fabrication de l'anion pentazolate par un procédé selon l'une quelconque des revendications 1 à 8, et le mélange de l'anion pentazolate ainsi fabriqué à un liant pour obtenir la composition énergétique.

## Patentansprüche

1. Verfahren zur Herstellung des Pentazolatanions, mindestens umfassend:
- eine Oxidation eines phenolischen Arylpentazols durch ein Oxidationsmittel mit hypervalentem Iod in Anwesenheit einer Base,
wobei das Oxidationsmittel für hypervalentes Iod von der allgemeinen Formel A-B ist,
wobei die Gruppierung A einen Benzolring bezeichnet und die Gruppierung B eine Struktur ist, umfassend ein hypervalentes Iodatom, das eine der zwei nachstehenden Formeln B1 oder B2 aufweist: 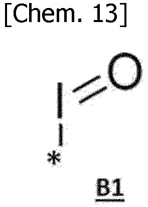 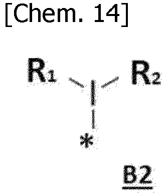
wobei *- in den Formeln B1 und B2 die Bindung des hypervalenten Iodatoms an den Benzolring A bezeichnet,
R₁ und R₂ in der Formel B2 gleich oder verschieden sind und unabhängig voneinander ausgewählt sind aus: **-OCOR, **-OR, wobei R eine geradkettige oder verzweigte Alkylkette umfassend 1 bis 4 Kohlenstoffatome bezeichnet, oder R₁ **-OH ist und R₂ **-OTs ist, wobei Ts eine Tosylgruppierung bezeichnet, wobei **- die Bindung des Sauerstoffatoms an das hypervalente Iodatom bezeichnet,
das phenolische Arylpentazol die folgende chemische Struktur aufweist: 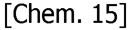 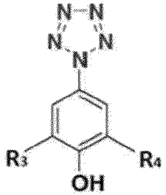
wobei R₃ und R₄ gleich oder verschieden sind und unabhängig voneinander ausgewählt sind aus: geradkettigen oder verzweigten Alkylketten, umfassend 1 bis 4 Kohlenstoffatome, unsubstituiert oder substituiert mit einer C₁- oder C₂-Alkoxygruppierung oder einem Dialkylamin.

2. Verfahren nach Anspruch 1, wobei die Oxidation in einem Lösungsmittel durchgeführt wird, umfassend Hexafluorisopropanol und mindestens eine Verbindung, ausgewählt aus Alkoholen oder Acetonitril.

3. Verfahren nach Anspruch 1 oder 2, wobei die Gruppierung B von Formel B1 ist.

4. Verfahren nach Anspruch 3, wobei die Gruppierung B von Formel B1 ist und die Oxidation in einem Lösungsmittel durchgeführt wird, umfassend Hexafluorisopropanol und mindestens eine Verbindung, ausgewählt aus Alkoholen oder Acetonitril.

5. Verfahren nach Anspruch 1 oder 2, wobei die Gruppierung B von Formel B₂ ist, wobei R₁ und R₂ gleich sind und jeweils **OCOMe oder **-OMe bezeichnen, wobei Me eine Methylgruppierung bezeichnet.

6. Verfahren nach einem der Ansprüche 1 bis 5, wobei die Base mindestens eine Verbindung umfasst, die ausgewählt ist aus: einem Alkalihydroxid, einem Erdalkalihydroxid, einem Metallhydroxid, Ammoniumhydroxid, einem quaternären Ammoniumhydroxid, einem Alkalicarbonat oder einem Gemisch dieser Verbindungen.

7. Verfahren nach Anspruch 6, wobei die Base Natriumhydroxid ist.

8. Verfahren nach einem der Ansprüche 1 bis 7, wobei die Oxidation in einem Lösungsmittel durchgeführt wird und wobei das Verfahren ferner nacheinander Folgendes umfasst:
- eine Entfernung des Lösungsmittels,
- eine Flüssig-Flüssig-Extraktion mittels eines Extraktionslösungsmittels, um die Reste des Oxidationsmittels zu entfernen
- eine Entfernung des Extraktionslösungsmittels, und
- eine selektive Extraktion des Pentazolatanions nach dieser Entfernung, wobei das Pentazolatanion selektiv in ein flüssiges Medium extrahiert wird, umfassend mindestens eines von Ethanol, Acetonitril oder Aceton oder ein Gemisch dieser Verbindungen.

9. Verfahren zur Herstellung einer energetischen Zusammensetzung, umfassend mindestens die Herstellung des Pentazolatanions durch ein Verfahren nach einem der Ansprüche 1 bis 8 und das Mischen des somit hergestellten Pentazolatanions mit einem Bindemittel, um die energetische Zusammensetzung zu erlangen.

## Claims

1. A method for producing the pentazolate anion, comprising at least:
- oxidation of a phenolic arylpentazole by a hypervalent iodine oxidant in the presence of a base,
said hypervalent iodine oxidant being of general formula A-B, wherein group A designates a benzene ring, and group B is a structure comprising a hypervalent iodine atom having one of the two formulas B1 or B2 below: 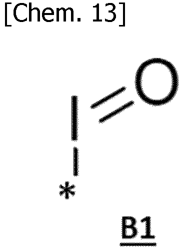 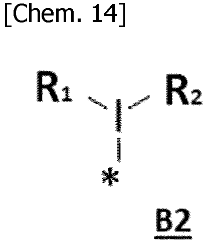
with *- designating, in the formulas B1 and B2, the bond of the hypervalent iodine atom to the benzene ring A,
in formula B2, R₁ and R₂ being identical or different and chosen independently of one another from: **-OCOR, **-OR, where R designates a linear or branched, alkyl chain comprising between 1 and 4 carbon atoms, or R₁ being **-OH and R₂ being **-OTs with Ts designating a tosyl group, where **- designates the bond of the oxygen atom to the hypervalent iodine atom,
the phenolic arylpentazole having the following chemical structure: 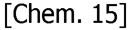 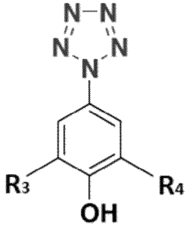
R₃ and R₄ being identical or different and chosen independently of one another from:
the linear or branched, alkyl chains comprising between 1 and 4 carbon atoms, not substituted or substituted by a C₁ or C₂ alkoxy group or by a dialkylamine.

2. The method according to claim 1, wherein the oxidation is carried out in a solvent comprising hexafluoroisopropanol and at least one compound chosen from alcohols or acetonitrile.

3. The method according to claim 1 or 2, wherein group B is of formula B1.

4. The method according to claim 3, wherein group B is of formula B1 and the oxidation is carried out in a solvent comprising hexafluoroisopropanol and at least one compound chosen from alcohols or acetonitrile.

5. The method according to claim 1 or 2, wherein group B is of formula B2 with R₁ and R₂ identical and each designating **-OCOMe or **-OMe, where Me designates a methyl group.

6. The method according to any one of claims 1 to 5, wherein the base comprises at least one compound chosen from: an alkali hydroxide, an alkaline-earth hydroxide, a metal hydroxide, ammonium hydroxide, a quaternary ammonium hydroxide, an alkali carbonate or a mixture of these compounds.

7. The method according to claim 6, wherein the base is sodium hydroxide.

8. The method according to any one of claims 1 to 7, wherein the oxidation is carried out in a solvent and wherein the method further comprises, successively:
- removal of the solvent,
- liquid-liquid extraction, using an extraction solvent, in order to remove the oxidant residues,
- removal of the extraction solvent, and
- selective extraction of the pentazolate anion after this removal, the pentazolate anion being selectively extracted in a liquid medium comprising at least one of ethanol, acetonitrile or acetone, or a mixture of these compounds.

9. A method for producing an energetic composition, comprising at least the production of the pentazolate anion by a method according to any one of claims 1 to 8, and mixing of the pentazolate anion thus produced with a binder in order to obtain the energetic composition.
